# EUROPEAN PATENT SPECIFICATION

(11) **EP 1 486 494 B1**
(45) Date of publication and mention of the grant of the patent: **14.07.2010**
(21) Application number: 03712691.9
(22) Date of filing: 13.03.2003
(51) Int. Cl.: C07D 333/62, C07D 413/12, C07D 417/14, A61K 31/381, A61K 31/422, A61K 31/427, A61P 9/00, A61P 9/10, A61P 9/12, A61P 11/06, A61P 13/12, A61P 17/02, A61P 17/06, A61P 27/02, A61P 29/00, A61P 35/00, A61P 37/08, A61P 43/00

(54) **N-SUBSTITUTED BENZOTHIOPHENESULFONAMIDE DERIVATIVE**
N-SUBSTITUIERTES BENZOTHIOPHENSULFONAMIDDERIVAT
DERIVE DU BENZOTHIOPHENESULFONAMIDE SUBSTITUE EN N

(30) Priority: 15.03.2002 JP 2002072307
(43) Date of publication of application: 15.12.2004
(73) Proprietor: TOA Eiyo Ltd., Chuo-ku Tokyo 104-0032 (JP)
(72) Inventor: SATOH, Shoji c/o Toa Eiyo Ltd. Tokyo Research Lab., Saitama-shi, Saitama 330-0834 (JP); MIZUNO, Yusuke c/o Toa Eiyo Ltd. Tokyo Research Lab., Saitama-shi, Saitama 330-0834 (JP); MASAKI, Hidekazu c/o Toa Eiyo Ltd., Saitama-shi, Saitama 330-0834 (JP)
(74) Representative: Albrecht, Thomas
(86) International application number: PCT/JP2003/003023
(87) International publication number: WO 2003/078419

(56) References cited:
- WO-A1-02/22595
- WO-A1-96/31492
- WO-A1-97/11941
- WO-A2-00/12623
- WO-A2-99/42465
- JP-A- 2000 095 770
- JP-A- 2001 097 946

## Description

### Field of the Invention

The present invention relates to medicaments, especially N-substituted benzothiophenesulfonamide derivatives or pharmaceutically acceptable salts thereof which selectively inhibit chymase, and chymase inhibitors containing the same as the active ingredient. Since the compounds have a selective inhibitory action on chymase, they are useful as agents for preventing or treating hypertension, hypercardia, cardiac infarction, arteriosclerosis, diabetic or non-diabetic renal diseases, diabetic retinopathy, restenosis after percutaneous transluminal coronary angioplasty (hereinafter, abbreviated as PTCA), intimal thickening after bypass grafting, chronic rheumatism, keloid, psoriasis, allergy, inflammation, asthma, atopic dermatitis and solid tumors caused by abnormal increase of production of angiotensin II (hereinafter, abbreviated as Ang II) or endothelin I (hereinafter, abbreviated as ET-1) based on chymase activity, or by activation of mast cell.

### Background Art

Since Ang II and ET-1 have a cell growth-accelerating action in addition to a blood pressure-elevating action, they are considered as causative agents or risk factors for diseases such as hypertension, hypercardia, cardiac infarction, arteriosclerosis, diabetic or non-diabetic renal diseases and restenosis after PTCA. Moreover, it is known that Ang II is formed from angiotensin I (hereinafter, abbreviated as Ang I) by angiotensin converting enzyme (hereinafter, abbreviated as ACE), and a large number of ACE inhibitors have been developed as agents for preventing or treating the above diseases. On the other hand, it is known that ET-1 is a physiologically active peptide composed of 21 amino acid residues (hereinafter, abbreviated as ET(1-21)) which is formed from big endothelin (hereinafter, abbreviated as Big ET-1) by endothelin converting enzyme (hereinafter, abbreviated as ECE), but ECE inhibitors and ET-1 receptor antagonists are still in developmental stages as medicaments.

Recently, it has been found that, in addition to ACE, an enzyme chymase derived from mast cell granules produces Ang II from Ang I. Urata et al. purified chymase from human heart and has shown that 70 to 80% amount of Ang II produced in heart and blood vessels was due to chymase (J. Biol. Chem., 265, 22348 (1990)). Moreover, when the fact that no effectiveness of ACE inhibitors on restenosis after PTCA is observed [MERCAPTOR study (Circulation, 86(1), 100 (1992)) and MARCAPTOR study (J. Am. Coll. Cardiol., 27(1), p. 1 (1996))] and the fact that chymase inhibitors are effective on a canine intimal thickening model of grafted blood vessel using jugular vein (Miyazaki, Takai et al.; Febs. Lett., 467, 141 (2000)) are together considered, it is important to inhibit chymase rather than ACE for preventing and treating cardiac and circulatory diseases caused by abnormal increase of the production of Ang II, and thus the application of chymase inhibitors to cardiac and circulatory diseases is suggested.

Furthermore, in the recent past, it has been revealed that chymase specifically degrades Big ET-1 into a physiologically active peptide composed of 31 amino acid residues (hereinafter, abbreviated as ET(1-31)). It has been reported that the ET(1-31) acts on the receptor on which original ET(1-21) acts, to cause bronchoconstriction and vasoconstriction (Kido et al.; J. Immunol., 159, 1987 (1997)). In this connection, with regard to the concentration in human blood, both of ET(1-31) and ET(1-21) have about the same distribution and activity, and after cardiac infarction, ET(1-31) increases more largely than ET(1-21) does, which is maintained for two weeks after the incidence (Tamaki, Nishisu et al.; Jpn. J. Pharmacol., 82(suppl I), 26 (2000)), and the fact suggests importance of inhibition of chymase and application of chymase inhibitors to cardiac and circulatory diseases.

Accordingly, chymase is considered to participate in production and degradation of physiologically active peptides, remodeling of extracellular matrix, network with cytokine, immunity, and the like and contribute to restoration of metabolic turnover. Thus, a chymase inhibitor is expected to apply to cardiac and circulatory diseases.

Moreover, as a result of administration of Ang II into a sponge in a hamster subdermally sponge-implanted model, removal of the sponge after 7 days, and measurement of hemoglobin content, vascularization was observed (mainly capillary vessels). When ovalbumin (10 µg/site/day) as an antigen is administered to a sensitized animal via sponge, vascularization occurs as in the case of Compound 48/80. This vascularization was also inhibited by chymostatin (Muramatsu et al.; J. Biol. Chem., 275(8), 5545 (2000)). The above results indicate that activation of mast cells by antigen stimulation can also cause vascularization, and chymase may be involved in this process. Thus, new roles of chymase are suggested in a variety of inflammatory allergy diseases. From such a viewpoint, a chymase inhibitor is expected to exhibit effects on solid tumors, diabetic retinopathy, rheumatoid arthritis and atherosclerosis.

Currently, as inhibitors against chymase, peptide-type chymase inhibitors are disclosed in JP-A-10-7661, JP-A-11-49739, JP-A-11-246437, WO98/09949, WO98/18794, WO99/45928, WO99/32459 and WO00/06594. On the other hand, non-peptide-type chymase inhibitors are disclosed in JP-A-10-87493, JP-A-10-245384, JP-A-12-95770, WO96/04248, WO97/11941, WO99/09977, WO00/03997, WO00/05204, WO00/10982, WO00/32587, WO01/32214, WO01/32621 and WO01/83471. However, until now, no clinically applicable chymase inhibitor has been found. Accordingly, it is desired to develop a clinically applicable chymase inhibitor which enables prevention and treatment of cardiac and circulatory diseases caused by abnormal increase of production of Ang II and ET-1.

### Disclosure of the Invention

As a result of the extensive studies for achieving the above objects, the present inventors have found that an N-substituted benzothiophenesulfonamide derivative or a pharmaceutically acceptable salt thereof has an excellent and selective human chymase inhibitory activity. Namely, the invention relates to an N-substituted benzothiophenesulfonamide derivative represented by the formula (I): 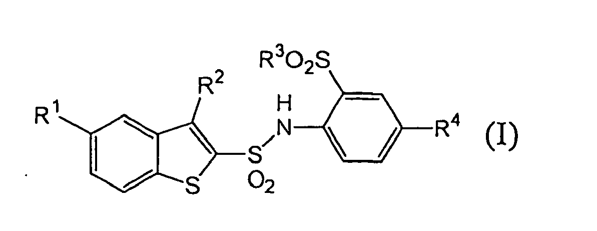 wherein R¹ represents a hydrogen atom, a halogen atom or a lower alkyl group having 1 to 4 carbon atoms; R² and R³ each may be the same or different and represents a lower alkyl group having 1 to 4 carbon atoms; and R⁴ represents a monocyclic heterocyclic group represented by the formula: 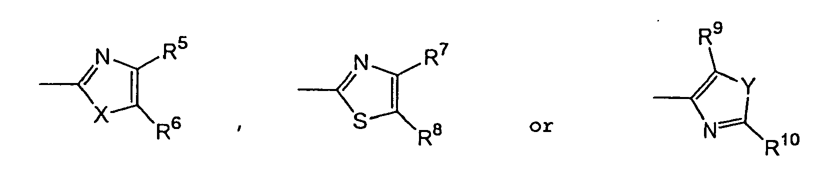 in which X represents an oxygen atom or NH, Y represents an oxygen atom, a sulfur atom or NH, R⁵ and R⁶ each may be the same or different and represents a hydrogen atom, a lower alkyl group having 1 to 4 carbon atoms which may be substituted by a halogen atom, a lower alkoxy group having 1 to 4 carbon atoms or a hydroxy lower alkyl group having 1 to 4 carbon atoms, R⁷ and R⁸ each may be the same or different and represents a hydrogen atom, a lower alkyl group having 1 to 4 lower alkoxy group having 1 to 4 carbon atoms, a hydroxy lower alkyl group having 1 to 4 carbon atoms, a lower alkoxycarbonyl group having 1 to 4 carbon atoms in the alkoxy residue or a carboxyl group, R⁹ and R¹⁰ each may be the same or different and represents a hydrogen atom, a lower alkyl group having 1 to 4 carbon atoms which may be substituted by a halogen atom, a lower alkoxy group having 1 to 4 carbon atoms, or a hydroxy lower alkyl group having 1 to 4 carbon atoms, provided that R⁵ and R⁶, R⁷ and R⁸, and R⁹ and R¹⁰ each are not hydrogen atoms at the same time, or a group represented by the formula: 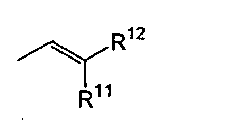 in which R¹¹ and R¹² each may be the same or different and represents a hydrogen atom, a lower alkyl group having 1 to 4 carbon atoms, a lower alkylsulfanyl group having 1 to 4 carbon atoms, a lower alkylsulfinyl group having 1 to 4 carbon atoms, a lower alkylsulfonyl group having 1 to 4 carbon atoms or a lower alkoxycarbonyl group having 1 to 4 carbon atoms in the alkoxy residue,
or a pharmaceutically acceptable salt thereof.

The N-substituted benzothiophenesulfonamide derivative represented by formula (I) or a pharmaceutically acceptable salt thereof according to the invention has a strong inhibitory activity against chymase and is a extremely useful compound for preventing or treating cardiac or circulatory diseases caused by abnormal increase of production of Ang II or ET-1 based on chymase activity, or by activation of mast cell.

Examples of the halogen atom for R¹ include a fluorine atom, a chlorine atom, a bromine atom or an iodine atom, and particularly, a fluorine atom or a chlorine atom is preferable.

Examples of the lower alkyl group for R¹, R², R³, R¹¹ and R¹² include a methyl group, an ethyl group, a propyl group, an isopropyl group, a butyl group, an isobutyl group, a sec-butyl group or a tert-butyl group, and particularly, a methyl group or an ethyl group is preferable.

Examples of the lower alkyl group which may be substituted by a halogen atom for R⁵, R⁶, R⁷, R⁸, R⁹ and R¹⁰ include a lower alkyl group or a lower alkyl group substituted by a halogen atom. Examples of the halogen atom include a fluorine atom, a chlorine atom, a bromine atom or an iodine atom, and examples of the lower alkyl group include a methyl group, an ethyl group, a propyl group, an isopropyl group, a butyl group, an isobutyl group, a sec-butyl group or a tert-butyl group. Examples of the lower alkyl group substituted by a halogen atom include a chloromethyl group, a bromomethyl group, a 1-chloroethyl group, and a chloromethyl group is preferable.

Examples of the lower alkoxy group for R⁵, R⁶, R⁷, R⁸, R⁹ and R¹⁰ include a methoxy group, an ethoxy group, a propoxy group, an isopropoxy group, a butoxy group, an isobutoxy group, a sec-butoxy group or a tert-butoxy group, and particularly, a methoxy group or an ethoxy group is preferable.

Examples of the hydroxy lower alkyl group for R⁵, R⁶, R⁷, R⁸, R⁹ and R¹⁰ include a hydroxymethyl group, a hydroxyethyl group, a hydroxypropyl group or a hydroxybutyl group, and particularly a hydroxymethyl group or a hydroxyethyl group is preferable.

Examples of the lower alkoxycarbonyl group for R⁷, R⁸, R¹¹ and R¹² include a methoxycarbonyl group, an ethoxycarbonyl group, a propoxycarbonyl group, an isopropoxycarbonyl group, a butoxycarbonyl group, an isobutoxycarbonyl group, a sec-butoxycarbonyl group or a tert-butoxycarbonyl group, and particularly, a methoxycarbonyl group or an ethoxycarbonyl group is preferable.

Examples of the lower alkylsulfanyl group for R¹¹ and R¹² include a linear or branched lower alkylsulfanyl group having 1 to 4 carbon atoms such as a methanesulfanyl group, an ethanesulfanyl group, a propanesulfanyl group or a butanesulfanyl group, and particularly, a methanesulfanyl group or an ethanesulfanyl group is preferable.

Examples of the lower alkylsulfinyl group for R¹¹ and R¹² include a linear or branched lower alkylsulfinyl group having 1 to 4 carbon atoms such as a methanesulfinyl group, an ethanesulfinyl group, a propanesulfinyl group or a butanesulfinyl group, and particularly, a methanesulfinyl group or an ethanesulfinyl group is preferable.

Examples of the lower alkylsulfonyl group for R¹¹ and R¹² include a linear or branched lower alkylsulfonyl group having 1 to 4 carbon atoms such as a methanesulfonyl group, an ethanesulfonyl group, a propanesulfonyl group or a butanesulfonyl group, and particularly, a methanesulfonyl group or an ethanesulfonyl group is preferable.

In this regard, examples of specific compounds include 2-[4-(5-fluoro-3-methylbenzo[b]thiophene-2-sulfonylamino)-3-methanesulfonylphenyl]thiazole-4-carboxylic acid, methyl 2-[4-(5-fluoro-3-methylbenzo[b]thiophene-2-sulfonylamino)-3-methanesulfonylphenyl]thiazole-4-carboxylate, 5-fluoro-N-[4-(4-hydroxymethylthiazol-2-yl)-2-methanesulfonylphenyl]-3-methylbenzo[b]thiophene-2-sulfonamide, N-[4-(4-chloromethylthiazol-2-yl)-2-methanesulfonylphenyl]-5-fluoro-3-methylbenzo[b]thiophene-2-sulfonamide, 5-fluoro-N-[2-methanesulfonyl-4-(4-methylthiazol-2-yl)phenyl]-3-methylbenzo[b]thiophene-2-sulfonamide, 5-fluoro-N-[2-methanesulfonyl-4-(2-methylthiazol-4-yl)phenyl]-3-methylbenzo[b]thiophene-2-sulfonamide, 5-fluoro-N-[2-methanesulfonyl-4-(5-methylthiazol-2-yl)phenyl]-3-methylbenzo[b]thiophene-2-sulfonamide, 5-fluoro-N-[2-methanesulfonyl-4-(5-methoxy-4-methylthiazol-2-yl)phenyl]-3-methylbenzo[b]thiophene-2-sulfonamide, 5-fluoro-N-[2-methanesulfonyl-4-(4,5-dimethylthiazol-2-yl)phenyl]-3-methylbenzo[b]thiophene-2-sulfonamide, 5-fluoro-N-[4-(4-hydroxymethyloxazol-2-yl)-2-methanesulfonylphenyl]-3-methylbenzo[b]thiophene-2-sulfonamide, N-[4-(4-chloromethyloxazol-2-yl)-2-methanesulfonylphenyl]-5-fluoro-3-methylbenzo[b]thiophene-2-sulfonamide, 5-fluoro-N-[2-methanesulfonyl-4-(4-methyloxazol-2-yl)phenyl]-3-methylbenzo[b]thiophene-2-sulfonamide, 5-fluoro-N-[2-methanesulfonyl-4-(5-methoxy-4-methyloxazol-2-yl)phenyl]-3-methylbenzo[b]thiophene-2-sulfonamide, 5-fluoro-N-[2-methanesulfonyl-4-(5-ethoxy-4-methyloxazol-2-yl)phenyl]-3-methylbenzo[b]thiophene-2-sulfonamide, 5-fluoro-N-[2-methanesulfonyl-4-(4,5-dimethyloxazol-2-yl)phenyl]-3-methylbenzo[b]thiophene-2-sulfonamide, 5-fluoro-N-[2-methanesulfonyl-4-(5-methyloxazol-2-yl)phenyl]-3-methylbenzo[b]thiophene-2-sulfonamide and 5-fluoro-N-[2-methanesulfonyl-4-((E)-2-methanesulfinyl-2-methylsulfanyl-vinyl)phenyl]-3-methylbenzo[b]thiophene-2-sulfonamide.

The following will describe the process for producing the N-substituted benzothiophenesulfonamide derivative or a pharmaceutically acceptable salt thereof of the invention. The compound of the formula (I) of the invention can be produced through the production process illustrated by the following reaction scheme.

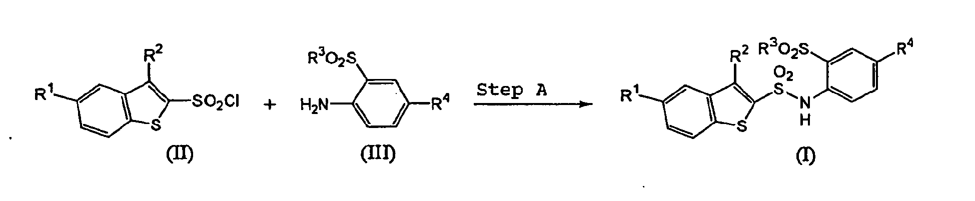

That is, the compound can be produced by reacting an amine represented by the compound (III) wherein R³ and R⁴ each has the same meaning as in the compound of the formula (I), with an sulfonyl chloride (II) wherein R¹ and R² each has the same meaning as in the compound of the formula (I), in the presence of a base such as sodium amide, lithium amide, sodium hydride, potassium carbonate, potassium tert-butoxide, triethylamine, ethyldiisopropylamine, pyridine, or 1,8-diazabicyclo[5.4.0]undec-7-ene (hereinafter, abbreviated as DBU) in a solvent such as dioxane, tetrahydrofuran (hereinafter, abbreviated as THF), acetone, dimethylformamide (hereinafter, abbreviated as DMF), dimethyl sulfoxide (hereinafter, abbreviated as DMSO), chloroform, pyridine or a mixed solvent thereof within the range of -10°C to a boiling point of the solvent.

In this connection, in the case of the compound of the formula (I) wherein R⁴ is a group represented by the formula: 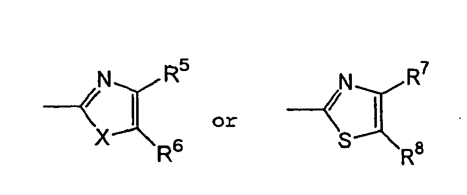 and R⁶ and R⁸ each has a substituent other than a hydrogen atom, the compound of the formula (I) can be also produced through the production process illustrated by the following reaction scheme.

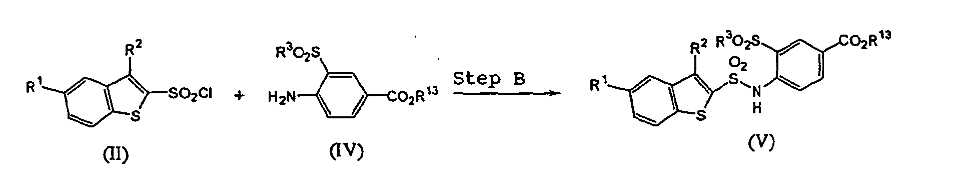

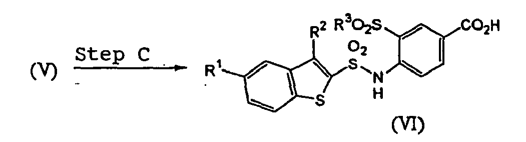

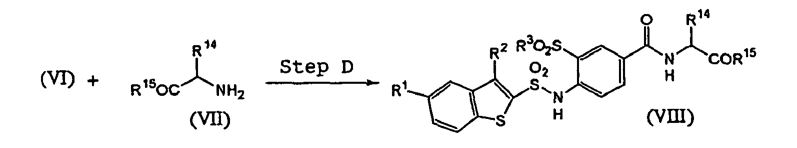

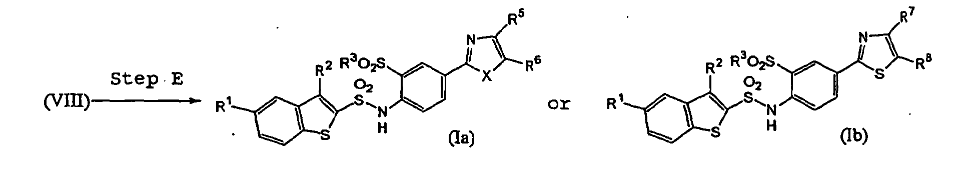

That is, the compound (V) is obtained by reacting an amine represented by the compound (IV) wherein R³ has the same meaning as in the compound of the formula (I) and R¹³ represents a lower alkyl group, synthesized from 4-chlorobenzoic acid according to the method known in a literature (J. Med. Chem., 40, 2017 (1997)), with an sulfonyl chloride (II) wherein R¹ and R² each has the same meaning as in the compound of the formula (I), in the presence of a base such as sodium amide, lithium amide, sodium hydride, potassium carbonate, potassium tert-butoxide, triethylamine, ethyldiisopropylamine, pyridine or DBU in a solvent such as dioxane, THF, acetone, DMF, DMSO, chloroform, pyridine or a mixed thereof within the range of -10°C to a boiling point of the solvent (Step B), and then ester hydrolysis was carried out to obtain a compound (VI) (Step C). Thereafter, the compound (VIII) is obtained by reacting the compound (VI) with an amine represented by the formula (VII) wherein R¹⁴ represents a hydrogen atom or a lower alkyl group and R¹⁵ represents a lower alkyl group or a lower alkoxy group, in the presence of a base such as triethylamine, ethyldiisopropylamine or DBU using a condensing agent such as N,N'-dicyclohexylcarbodiimide (hereinafter, abbreviated as DCC) or 1-ethyl-3-(3-dimethylaminopropyl)carbodiimide (hereinafter, abbreviated as EDC) (Step D), and then a compound (Ia) and (Ib) are obtained by using phosphorus oxychloride or diphosphorus pentasulfide (Step E), whereby the production is completed.

In addition, in the case of the compound of the formula (I) wherein R⁴ is a group represented by the formula: 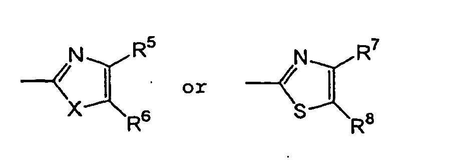 and R⁶ and R⁸ each is a hydrogen atom, the compound of the formula (I) can be also produced through the production process illustrated by the following reaction scheme.

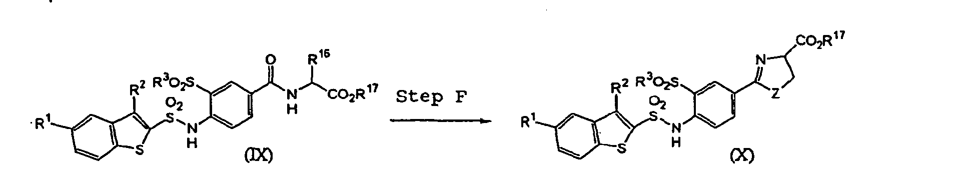

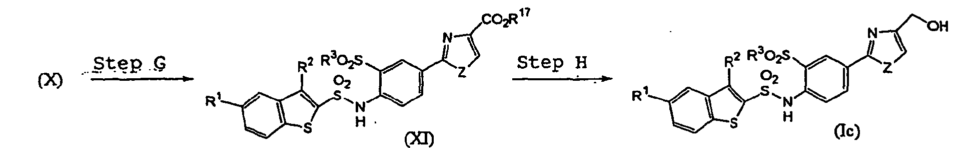

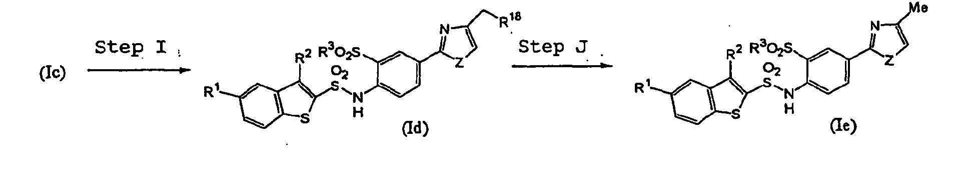

That is, a compound (XI) wherein Z represents an oxygen atom, a sulfur atom or NH, is obtained in accordance with the method known in literatures (Tetrahedron Letters, 33, 907 (1992), J. Org. Chem., 38, 26 (1973), J. Org. Chem., 58, 4494 (1993), Org. Lett., 2, 1165 (2000), tetrahedron Letters, 42, 4171 (2001)) starting with a compound (IX) wherein R¹, R² and R³ each has the same meaning as in the compound of the formula (I), R¹⁶ represents a hydroxymethyl group or a mercaptomethyl group which may have a protective group such as a trityl group and R¹⁷ represents a lower alkyl group (Steps F and G). The compound (IX) is obtained by reacting the compound (VI) with serine ester hydrochloride, cysteine ester hydrochloride, S-tritylcysteine ester or the like in the presence of a base such as triethylamine, ethyldiisopropylamine or DBU using a condensing agent such as EDC. A compound (Ic) can be produced by further converting the ester group into a hydroxymethyl group through reduction (Step H). Also, a compound (Id) wherein R¹⁸ represents a halogen atom can be produced by halogenating the compound (Ic) (Step I), and a compound (Ie) can be further produced by reducing the halogen atom of the compound (Id) to form a methyl group (Step J).

In this connection, a compound (Ig) can be produced by subjecting a compound (If), i.e., a compound of the compound (XI) in which Z is a sulfur atom, wherein R¹, R² and R³ each has the same meaning as in the compound of the formula (I) and R¹⁹ represents a lower alkyl group, to ester hydrolysis (Step K).

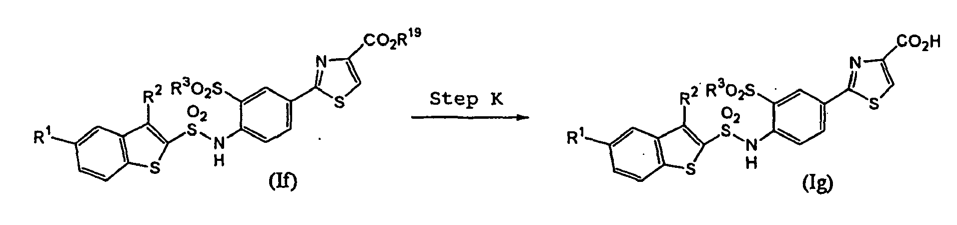

Also, in the case of the compound of the formula (I) wherein and R⁴ is a group represented by the formula: 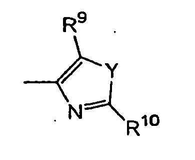 the compound of formula (I) can be also produced through the following production process illustrated by the following reaction scheme.

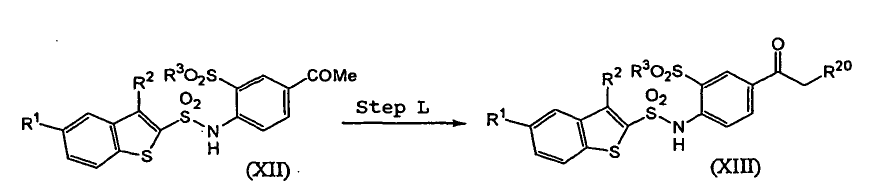

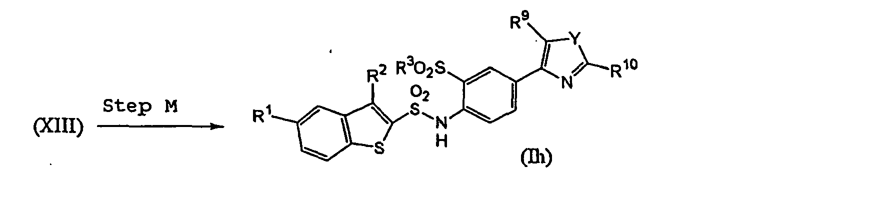

That is, a compound (XIII) wherein R¹, R² and R³ each has the same meaning as in the compound of the formula (I) and R²⁰ represents a halogen atom, is obtained from the compound (XII) wherein R¹, R² and R³ each has the same meaning as in the compound of the formula (I), in accordance with the method known in a literature (JP-A-2000-256262) (Step L). Further, a compound (Ih) can be produced by subjecting the compound (XIII) to ring closure with thioacetamide or formamide (Step M).

In this connection, in the case of the compound of formula (I) wherein R⁴ is a group represented by the formula: 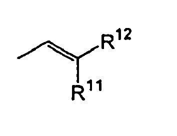 the compound can be also produced through the following production process illustrated by the following reaction scheme.

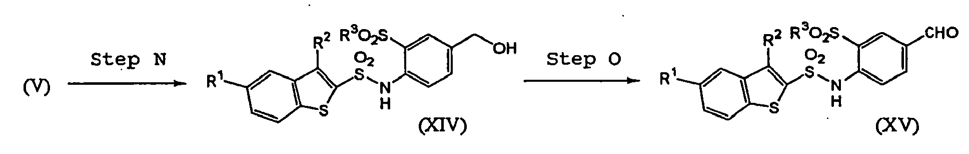

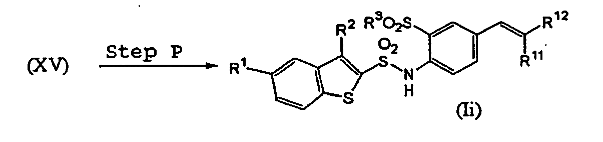

That is, a compound (XV) is obtained by reducing the ester group of the compound (V) wherein R¹, R² and R³ each has the same meaning as in the compound of the formula (I) and R¹³ represents a lower alkyl group, followed by oxidation to form an aldehyde group (Steps N and O) and then a compound (Ii) wherein R¹, R² and R³ each has the same meaning as in the compound of the formula (I), and R¹¹ and R¹² has the same meaning as above, can be produced in accordance with the method known in a literature (Bull. Chem. Soc. Jpn., 52, 2013 (1979)) (Step P).

The thus formed compound of formula (I) can be isolated and purified by conventional methods such as recrystallization and column chromatography.

The compound of formula (I) of the invention can be formed into a pharmaceutically acceptable salt with an acid or base, e.g., a salt with an inorganic acid such as hydrochloride, hydrobromide, hydroiodide, sulfate, nitrate or phosphate; a salt with an organic acid such as acetate, trifluoroacetate, oxalate, fumarate, maleate, tartrate, mesylate or tosylate; a salt with an alkali metal such as sodium salt or potassium salt; or a salt with an alkaline earth metal such as calcium salt depending on the compound, by a usual method.

The compound of formula (I) of the invention includes hydrates and various solvates. All the crystal forms are also encompassed within the compound of formula (I).

The compound of the formula (I) or a pharmaceutically acceptable salt thereof may be administered orally or parenterally, e.g., via intravenous or intramuscular injection.

Examples of preparations for oral administration include tablets including sugar-coated tablets and film-coated tablets, pills, granules, powders, capsules including soft capsules, syrups, emulsions, suspensions, and the like.

The preparations for oral administration can be manufactured with mixing additives usually employed in the pharmaceutical field in accordance with a known method. Examples of such additives include an excipient such as lactose, mannitol or anhydrous calcium hydrogen phosphate; a binder such as hydroxypropyl cellulose, methyl cellulose or polyvinylpyrrolidone; a disintegrator such as starch and carboxymethyl cellulose; a lubricant such as magnesium stearate or talc; and the like.

Parenteral administration may be conducted as injections and the like. These injections can be manufactured by a known method, e.g., dissolving the compound of formula (I) or a pharmaceutically acceptable salt thereof in Japanese Pharmacopoeia-grade water for injection. As needed, an isotonic agent such as sodium chloride, a buffering agent such as sodium dihydrogen phosphate or sodium monohydrogen phosphate, or the like may be mixed.

The dose of the compound of the formula (I) or a pharmaceutically acceptable salt thereof per day for an adult may vary depending on the condition, body weight and age of an patient, kind of the compound, and administration route, but is suitably from about 0.01 mg to 1,000 mg, preferably about 0.1 mg to 300 mg in the case of oral administration. In the case of parenteral administration, the dose may be from one tenth to a half of the dose in the case of oral administration. The dose can be appropriately changed depending on the condition, body weight, age and the like of an individual patient.

### Best Mode for Carrying Out the Invention

The following will describe the invention in more detail with reference to Reference Examples and Examples, but the invention is not limited thereto.

### Reference Example 1

### Methyl 4-(5-fluoro-3-methylbenzo[b]thiophene-2-sulfonylamino)-3-methanesulfonylbenzoate

Into 300 mL of THF was dissolved 14.0g of methyl 4-amino-3-methanesulfonylbenzoate, followed by addition of 6.10 g of sodium hydride (oily, 60%) at 0°C. After 40 minutes of stirring at the same temperature, 16.0 g of 2-chlorosulfonyl-5-fluoro-3-methylbenzo[b]thiophene was added at 0°C, followed by 3 hours of stirring at room temperature. After confirmation of disappearance of the starting material, the reaction was terminated by adding 2 mol/L hydrochloric acid at 0°C, followed by extraction with ethyl acetate. The organic layer was washed with saturated aqueous sodium hydrogen carbonate solution and saturated brine successively, and then dried over anhydrous sodium sulfate. The solvent was removed by evaporation and the residue was diluted with ethyl acetate. After the solution was treated with active carbon, purification by recrystallization (ethyl acetate/ether = 3/1) afforded 24.8 g of the title compound as colorless powder.
Melting point: 202-204°C
¹H-NMR (CDCl₃) : δ 2.69 (3H,s) ,3.06 (3H,s) ,3.90 (3H,s) ,7.2 8 (1H,ddd,J=2.6,8.7,8.9Hz) ,7.46 (1H,dd,J=2.6,9.2Hz) ,7.76
(1H,dd,J=4.7,8.9Hz) ,7.87 (1H,d,J=8.8Hz) ,8.19 (1H,dd,J=2. 0,8.8Hz) ,8.50 (1H,d,J=2.0Hz) ,9.83 (1H,s) .
IR νₘₐₓ (KBr) :3182,1724,1604,1504,1442,1396,1346,1303, 1157 cm⁻¹.

### Reference Example 2

### [4-(5-Fluoro-3-methylbenzo[b]thiophene-2-sulfonylamino)-3-methanesulfonyl]benzoic acid

Into 500 mL of methanol was dissolved 24.8 g of the compound of Reference Example 1, and 50 mL of 1 mol/L sodium hydroxide was added thereto. After 3 hours of stirring under heating and refluxing, the solvent was removed by evaporation under reduced pressure and water was added to the resulting residue. The mixture was washed with ether. To the aqueous layer was added 2 mol/L hydrochloric acid, followed by extraction with ethyl acetate. The organic layer was washed with saturated brine and then dried over anhydrous sodium sulfate. The solvent was removed by evaporation under reduced pressure and the resulting residue was washed with ether to obtain 14.3 g of the title compound as colorless powder.
Melting point: 290°C
¹H-NMR (CDCl₃) : δ 2.66 (3H,s) ,3.05 (3H,s) ,6. 76 (1H,dd,J=2 .0,8.6Hz) ,7.45 (1H,dd,J=2.0,8.6Hz) ,7.75 (1H,dd,J=4.6,9.0H z) ,7.82 (1H,d,J=8.8Hz) ,8.17 (1H,dd,J=2.0,8.8Hz) ,8.49 (1H ,d,J=2.0Hz) .
IR νₘₐₓ (KBr) :3239,2925,1687,1609,1501,1442,1421,1400,13 56,1287,1161 cm⁻¹.

### Reference Example 3

### Methyl (2S)-2-[4-(5-fluoro-3-methylbenzo[b]thiophene-2-sulfonylamino)-3-methanesulfonylphenylcarboxyamido]propionate

Into 100 mL of dichloromethane was suspended 3.78 g of L-alanine methyl ester hydrochloride, and 3.8 mL of triethylamine was added at 0°C. Thereto was added 100 mL of dichloromethane suspension of 10.0 g of the compound of Reference Example 2. After 5 minutes of stirring at the same temperature, 5.19 g of EDC hydrochloride was added and the mixture was stirred at room temperature for 19 hours. After the reaction was terminated with 1 mol/L hydrochloric acid, the dichloromethane layer was separated. After removal of the solvent by evaporation under reduced pressure, a mixed solution of ethyl acetate-THF (1/1) was added to the resulting residue, followed by successive washing with water and saturated brine and drying over anhydrous magnesium sulfate. The solvent was removed by evaporation under reduced pressure and the resulting residue was washed with methanol to obtain 9.22 g of the title compound as colorless powder. Melting point: 162-163°C
¹H-NMR (CDCl₃) : δ 1.49 (3H, d, J=7.3Hz) , 2.69 (3H, s) , 3 .07 (3H, s) , 3.79 (3H, s) , 4.74 (1H,dq,J=7.3,7.3Hz) , 6. 96 (1H,d,J=7.3Hz) , 7.28 (1H,ddd,J=2.4,8.6,8.8Hz) ,7.47 (1 H,dd,J=2.4, 9.2Hz) , 7.77 (1H,dd,J=4.7,8.8Hz) , 7.81 (1H,d ,J=8.7Hz) , 7.89 (1H,dd,J=2.0,8.7Hz) , 8.25 (1H,d,J=2.0Hz ) , 9.71 (1H,s) .
IR νₘₐₓ (KBr) :3323,3222,3068,3003,2924,1737,1636,1607,14 96,1306,1165,924 cm⁻¹.

### Reference Example 4

### Methyl (4R)-2-[4-(5-fluoro-3-methylbenzo[b]thiophene-2-sulfonylamino)-3-methanesulfonylphenyl]thiazoline-4-carboxylate

Into 1500 mL of dichloromethane was dissolved 31.04 g of methyl (2R)-2-[4-(5-fluoro-3-methylbenzo[b]thiophene-2-sulfonylamino)-3-methanesulfonylphenylcarboxyamido]-3-tritylthiopropionate. After the solution was cooled to 0°C, 19.8 mL of hexamethylphosphoramide was added and 435 mL of dichloromethane solution of 12.9 mL of titanium tetrachloride was added dropwise. After 21 hours of stirring at room temperature, the reaction was terminated by adding water. The solvent was removed by evaporation under reduced pressure and the resulting residue was redissolved into ethyl acetate. The solution was washed with water and saturated brine, successively. After drying over anhydrous sodium sulfate, the solvent was removed by evaporation under reduced pressure and the resulting residue was purified by silica gel column chromatography (chloroform) and then recrystallized from chloroform-hexane (3/1) to obtain 11.09 g of the title compound as colorless powder.
Melting point: 170-171°C
¹H-NMR (CDCl₃) : δ 2.68 (3H, s) , 3.03 (3H, s) , 3.66 (1H,dd,J=9.1,11.5Hz) , 3.73 (1H,dd,J=9.1, 11.5Hz) , 3.82 (3H,s) , 5.25 (1H,t,J=9.1Hz) , 7.28 (1H,ddd,
J=2.5,8.7,8.9Hz) , 7.46 (1H,dd,J=2.5, 9.2Hz) , 7.76 (1H,dd,J=4.7,8.9Hz) , 7.85 (1H,d,J=8.6Hz) , 8.01 (1H,dd,J=2.0,8.6Hz) , 8.31 (1H,d,J=2.0Hz) , 9.76 (1H,s) .
IR νₘₐₓ (KBr) : 3186,3029,3000,2954,2920,1744,1606,1498, 1357,1293,1225,1163,1131,989,927 cm⁻¹.

### Reference Example 5

### 5-Fluoro-N-(4-hydroxymethyl-2-methanesulfonylphenyl)-3-methylbenzo[b]thiophene-2-sulfonamide

Into 120 mL of toluene was dissolved 2.08 g of the compound of Reference Example 1. After cooling to - 30°C, 22.5 mL of 1.01 mol/L toluene solution of diisobutylaluminum hydride was added thereto. After 5 hours of stirring at the same temperature, water was added to the reaction solution to terminate the reaction and then the mixture was diluted with ethyl acetate, followed by addition of saturated aqueous potassium sodium tartrate solution and 30 minutes of stirring at room temperature. The mixture was extracted with ethyl acetate and the organic layer was washed with saturated brine and then dried over anhydrous sodium sulfate. The solvent was removed by evaporation under reduced pressure and the resulting residue was purified by silica gel column chromatography (ethyl acetate/hexane = 1/1) to obtain 1.38 g of the title compound as colorless powder. Melting point: 120-121°C
¹H-NMR (CDCl₃) : δ 2.68 (3H,s) , 2.96(3H,s), 4.68(2H,s), 7 .26(1H,ddd,J=2.4,8.7,9.0Hz), 7.46 (1H,dd,J=2.4,9.0Hz) , 7. 57 (1H,dd,J=1.8,8.4Hz) ,7.75 (1H,dd,J=4.5,8.7Hz ,7.77 (1H, d,J=8.4Hz) , 7.86(1H,d,J=1.8Hz), 9.48(1H,s).
IR νₘₐₓ (KBr) : 3504,3221,1608,1497,1347,1296,1150,926 cm⁻¹.

### Reference Example 6

### 4-(5-Fluoro-3-methylbenzo[b]thiophene-2-sulfonylamino)-3-methanesulfonyl]benzaldehyde

Into 25 mL of ethyl acetate was dissolved 830 mg of the compound of Reference Example 5, and 4.15 g of active manganese dioxide was added thereto, followed by 5 hours of stirring at room temperature. The reaction solution was diluted with ethyl acetate and then was filtered through celite to remove manganese dioxide. The solvent was removed by evaporation under reduced pressure to obtain 696 mg of the title compound as colorless powder. Melting point: 167-170°C

¹H-NMR (CDCl₃) : δ 2.70 (3H,s) , 3.10 (3H, s) , 7.31 (1H, dd d, J=2.4, 8.7, 9.0Hz), 7.48 (1H, dd, J=2.4, 9.0Hz) , 7.78 (1H, dd J=4.5, 9.0Hz) , 7.96 (1H, d, J=8.7Hz) , 8.06(1H, d d, J=2.1, 8.7Hz) , 8.36(1H, d, J=2.1Hz), 9.90(1H, s), 9.92 (1H, s).
IR νₘₐₓ (KBr) : 3260,1694,1602,1496,1308,1164,912 cm⁻¹.

### Example 1

### 5-Fluoro-N-[2-methanesulfonyl-4-(5-methoxy-4-methylthiazol-2-yl)phenyl]-3-methylbenzo[b]thiophene-2-sulfonamide (Compound 1)

Under an argon atmosphere, 41 mg of 2-methanesulfonyl-4-(5-methoxy-4-methylthiazol-2-yl)aniline was dissolved into a mixed solution of 3 mL of THF and 3 mL of dimethylacetamide. The solution was cooled to -25°C and 15 mg of sodium hydride (oily, 60%) was added thereto, followed by 10 minutes of stirring at the same temperature. Further, 44 mg of 2-chlorosulfonyl-5-fluoro-3-methylbenzo[b]thiophene was added and the whole was stirred for 2 hours at the same temperature, followed by termination of the reaction with 1 mol/L hydrochloric acid. After the reaction mixture was warmed to room temperature, the mixture was extracted with ethyl acetate-toluene (2/1) and the organic layer was washed with water and saturated brine, successively. After the organic layer was dried over anhydrous sodium sulfate, the solvent was removed by evaporation under reduced pressure and the resulting residue was purified by silica gel column chromatography (ethyl acetate/hexane = 1/1) to obtain 51 mg of the title compound as light yellow powder. Melting point: 216-217°C
¹H-NMR (CDCl₃) : δ 2.29 (3H,s) , 2.68 (3H,s) , 2.99 (3H,s ) , 3.93 (3H,s) , 7.27 (1H,ddd,J=2.6,8.6, 8.8Hz) ,7.46 (1 H,dd,J=2.6,9.2Hz) ,7.75 (1H,dd,J=4.7,8.8Hz) ,7.82 (1H,d, J= 8.7Hz) ,7.96 (1H,dd,J=2.1,8.7Hz) ,8.25 (1H,d,J=2.1Hz) ,9.5 7 (1H,s) .
IR νₘₐₓ (KBr) 3202,2989,2910,1604,1558,1501,1441,1349,12 98,1253,1156,1133,926 cm⁻¹.

### Example 2

### 5-Fluoro-N-[2-methanesulfonyl-4-(5-methoxy-4-methyloxazol-2-yl)phenyl]-3-methylbenzo[b]thiophene-2-sulfonamide (Compound 2)

Into 26 mL of phosphorus oxychloride was added 5.28 g of the compound of Reference Example 3, followed by 3 hours of heating under refluxing. After the reaction mixture was cooled to room temperature, the mixture was poured into ice and extracted with chloroform. The organic layer was washed with water and saturated brine, successively and dried over anhydrous sodium sulfate. The solvent was removed by evaporation under reduced pressure and the resulting residue was purified by silica gel column chromatography (chloroform) to obtain 2.02 g of the title compound as colorless powder. Melting point: 232-233°C
¹H-NMR (DMSO-d₆) : δ2.02 (3H,s) , 2.57 (3H,s) , 3.32 (3H,s) , 3.96 (3H,s) , 7.46 (1H, ddd, J=2.5,9.0,9.0Hz) , 7.54 (1H, d,J=8.4Hz) , 7.81 (1H,dd,J=2.5,9.9Hz) , 8.06 (1H,dd,J=1.9,8 .4Hz) , 8.10 (1H,dd,J=4.9,9.0Hz) , 8.26 (1H,d,J=1.9Hz) .
IR νₘₐₓ (KBr) : 3253,3083,3000,2922,1665,1491,1442,1393,13 54,1308,1169,1131 cm⁻¹.

### Example 3

### 5-Fluoro-N-[2-methanesulfonyl-4-(5-methylthiazol-2-yl)phenyl]-3-methylbenzo[b]thiophene-2-sulfonamide (Compound 3)

Into 5 mL of 1,4-dioxane solution of 150 mg of 4-(5-fluoro-3-methylbenzo[b]thiophene-2-sulfonylamino)-3-methanesulfonyl-N-(2-oxopropyl)benzamide was added 135 mg of diphosphorus pentasulfide, followed by 4.5 hours of heating under refluxing. The reaction was terminated by adding water to the reaction mixture and the mixture was extracted with ethyl acetate. The organic layer was washed with water and saturated brine, successively and then dried over anhydrous sodium sulfate. The solvent was removed by evaporation under reduced pressure and the resulting residue was purified by silica gel column chromatography (hexane/ethyl acetate = 2/1 to 1/1) to obtain 115 mg of the title compound as amorphous.
¹H-NMR (CDCl₃) : 2.51 (3H,s), 2.69 (3H,s), 3.02 (3H,s), 7. 27 (1H,ddd,J=2.4,8.6,8.9Hz), 7.47 (1H,dd,J=2.4,9.2Hz), 7. 50 (1H,s), 7.76 (1H,dd,J=4.8,8.9Hz), 7.86 (1H,d,J=8.8Hz),
8.05 (1H,dd,J=2.1,8.8Hz), 8.35 (1H,d,J=2.1Hz), 9.64 (1H, s).
IR νₘₐₓ (KBr) : 3240,3010,2926,1607,1499,1353,1302,1160,99 4 cm⁻¹.

In the following, the compounds 4 to 8 of Examples 4 to 8 were synthesized in the same manner as in Examples 2 and 3.

**Table 1**

| | | | | |
|---|---|---|---|---|
| 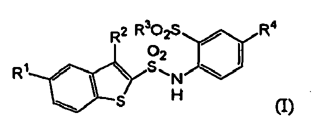 | | | | |

| Example | R¹ | R² | R³ | R⁴ |
|---|---|---|---|---|
| 4 | Cl | Me | Me | 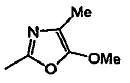 |
| 5 | F | Me | Me | 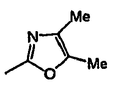 |
| 6 | F | Me | Me | 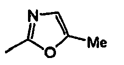 |
| 7 | F | Me | Me | 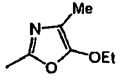 |
| 8 | F | Me | Me | 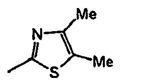 |

### Example 9

### 5-Fluoro-N-[4-(4-hydroxymethylthiazol-2-yl)-2-methanesulfonylphenyl]-3-methylbenzo[b]thiophene-2-sulfonamide (Compound 9)

Into 226 mL of THF suspension of 298 mg of lithium aluminum hydride was added dropwise 452 mL of THF solution of 4.52 g of Compound 15, followed by 6 hours of stirring at room temperature. Further, 298 mg of lithium aluminum hydride was added thereto, followed by 14 hours of stirring at room temperature. After confirmation of disappearance of the starting material, the reaction was terminated by adding 10 mL of water at 10°C and the mixture was stirred for 30 minutes. The solvent was removed by evaporation under reduced pressure and chloroform was added to the resulting residue. The organic layer was washed with 1 mol/L sulfuric acid, water and saturated brine, successively and then dried over anhydrous sodium sulfate. The solvent was removed by evaporation under reduced pressure and the resulting residue was purified by silica gel column chromatography (ethyl acetate/hexane = 1/1). To the resulting crystals were added 140 mL of chloroform and 28 mL of hexane, followed by heating under refluxing. After filtration at a hot state, the crystals obtained by recrystallization were collected by filtration and dried under reduced pressure to obtain 2.58 g of the title compound as light yellow powder.
Melting point: 209-210°C
¹H-NMR (CDCl₃) : δ 2.69 (3H,s) ,3.04 (3H,s) ,4.80 (2H,s) ,7. 22 (1H,s) ,7.27 (1H,ddd,J=2.4,8.6,8.8Hz) ,7.47 (1H,dd,J=2.4 ,9.2Hz) ,7.76 (1H,dd,J=4.7,8.8Hz) ,7.88 (1H, d, J=8.8Hz) ,8.0 9 (1H,dd,J=2.2,8.8Hz) ,8.42 (1H,d,J=2.2Hz) ,9.65 (1H,s) .
IR νₘₐₓ (KBr) : 3423,3237,3114,3026,2930,1605,1509,1445,13 54,1294,1152,1135 cm⁻¹.

### Example 10

### N-[4-(4-chloromethylthiazol-2-yl)-2-methanesulfonylphenyl]-5-fluoro-3-methylbenzo[b]thiophene-2-sulfonamide (Compound 10)

Into 20 mL of chloroform was suspended 159 mg of Compound 9, and 47 µL of thionyl chloride was added at 0°C, followed by 21 hours of stirring at room temperature. Further, 1 mL of thionyl chloride was added, followed by 1 hour of heating under refluxing. After 2 mL of triethylamine was added at 0°C and the mixture was stirred for 3 hours, the reaction was terminated with 1 mol/L hydrochloric acid, followed by extraction with ethyl acetate. The organic layer was washed with water and saturated brine, successively and then dried over anhydrous sodium sulfate. The solvent was removed by evaporation under reduced pressure and the resulting residue was purified by silica gel column chromatography (chloroform) to obtain 104 mg of the title compound as light brown powder.
Melting point: 190-191°C
¹H-NMR (CDCl₃) : δ 2.69 (3H,s) , 3.04 (3H,s) , 4.70 (2H,s) , 7.28 (1H,ddd,J=2.5,8.6,8.8Hz) , 7.34 (1H,s) , 7.47 (1H,dd,J =2.5,9.2Hz) , 7.76 (1H,dd,J=4.7,8.8Hz) , 7.89 (1H,d,J=8.8Hz ) ,8.11 (1H, dd, J=2.2, 8.8Hz) ,8.41 (1H,d,J=2.2Hz) ,9.66 (1H,s) .
IR νₘₐₓ (KBr) :3236,3098,3027,2927,1607,1507,1457,1354,13 06,1156,1137,912 cm⁻¹.

### Example 11

### 5-Fluoro-N-[2-methanesulfonyl-4-(4-methylthiazol-2-yl)phenyl]-3-methylbenzo[b]thiophene-2-sulfonamide (Compound 11)

Into 10 mL of acetone was dissolved 64 mg of Compound 10, and 180 mg of sodium iodide was added, followed by 24 hours of heating under refluxing. Further, 180 mg of sodium iodide was added and the mixture was heated under refluxing for 19 hours. The solvent was removed by evaporation under reduced pressure and the residue was dissolved into ethyl acetate. The solution was washed with water, 5% aqueous sodium thiosulfate solution, water and saturated brine, successively, and then dried over anhydrous sodium sulfate. After the solvent was removed by evaporation under reduced pressure, the resulting 5-fluoro-N-[4-(4-iodomethylthiazol-2-yl)-2-methanesulfonylphenyl]-3-methylbenzo[b]thiophene-2-sulfonamide was dissolved into a mixed solution of 5 mL of toluene and 0.5 mL of DMSO, and 45 µL of tributyltin hydride and 13 µL of 1.06 mol/L hexane solution of triethylboron were added, followed by 6 hours of stirring at room temperature. Further, 24 µl of tributyltin hydride was added and the mixture was stirred at room temperature for 3 hours. Then, the reaction was terminated by adding saturated aqueous ammonium chloride solution, followed by extraction with ethyl acetate. The organic layer was washed with water and saturated brine, successively, and then dried over anhydrous sodium sulfate. The solvent was removed by evaporation under reduced pressure and the resulting residue was purified by silica gel column chromatography (hexane to hexane/ethyl acetate = 3/1) to obtain 29 mg of the title compound as colorless powder.
Melting point: 199-200°C
¹H-NMR (CDCl₃) : δ 2.48 (3H,s) , 2.69 (3H,s) , 3.03 (3H,s) , 6.91 (1H,s) , 7.27 (1H,ddd,J=2.4,8.6,8.8Hz) , 7.46 (1H,dd,J =2.4,9.2Hz) , 7.75 (1H,dd,J=4.7,8.8Hz) , 7.87 (1H,d,J=8.7Hz ) , 8.09 (1H,dd,J=2.1,8.7Hz) , 8.40 (1H,d,J=2.1Hz) , 9.65 (1H,s) .
IR νₘₐₓ (KBr) :3243,3105,3028,2925,1607,1508,1442,1355,13 08,1162,1135,913 cm⁻¹.

In the following, Compounds 12 to 14 of Examples 12 to 14 were synthesized in the same manner in Examples 9 to 11.

**Table 2**

| Example | R¹ | R² | R³ | R⁴ |
|---|---|---|---|---|
| 12 | F | Me | Me | 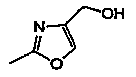 |
| 13 | F | Me | Me | 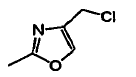 |
| 14 | F | Me | Me | 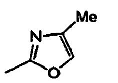 |

### Example 15

### Methyl 2-[4-(5-fluoro-3-methylbenzo[b]thiophene-2-sulfonylamino)-3-methanesulfonylphenyl]thiazole-4-carboxylate (Compound 15)

Into 204 mL of dichloromethane was dissolved 11.06 g of the compound of Reference Example 4, followed by dropwise addition of 2.4 mL of bromotrichloromethane at 0°C over the period of 5 minutes. After 20 minutes of stirring at the same temperature, 7.6 mL of DBU was added dropwise over the period of 15 minutes. After the mixture was stirred at room temperature for 5 hours, the reaction was terminated with 1 mol/L hydrochloric acid. The organic layer was separated, washed with water and saturated brine, and then dried over anhydrous sodium sulfate. The solvent was removed by evaporation under reduced pressure and the resulting residue was purified by silica gel column chromatography (chloroform) to obtain 9.18 g of the title compound as colorless powder. Melting point: 241-242°C
¹H-NMR (DMSO-d₆) : δ 2.61 (3H,s) , 3.38 (3H,s) , 3.87 (3H,s ) ,7,47 (1H,ddd,J=2.5,9.0,9.0Hz) , 7.60 (1H,d,J=8.6Hz) , 7. 83 (1H,dd,J=2.5,9.9Hz) , 8.11 (1H,dd,J=4.7,9.0Hz) , 8.22 (1 H,dd,J=2.1, 8.6Hz) , 8.44 (1H,d,J=2.1Hz) , 8.63 (1H,s) .
IR νₘₐₓ (KBr) : 3193,3113,3021,3003,2923,1730,1606,1509,1 392,1359,1295,1225,1162,1131,988,918 cm⁻¹.

### Example 16

### 2-[4-(5-Fluoro-3-methylbenzo[b]thiophene-2-sulfonylamino)-3-methanesulfonylphenyl]thiazole-4-carboxylic acid (Compound 16)

Into 45 mL of methanol was dissolved 954 mg of Compound 15, and 4.0 mL of 1 mol/L sodium hydroxide was added thereto. After 20 minutes of stirring under heating and refluxing, the solvent was removed by evaporation under reduced pressure, and the resulting residue was extracted with ether-water (1/1). To the aqueous layer was added 2 mol/L hydrochloric acid, and the precipitated crystals were washed with ether to obtain 746 mg of the title compound as colorless powder. Melting point: 257-258°C
¹H-NMR (DMSO-d₆) : δ 2.59 (3H,s) ,3.36 (3H,s) , 7.45 (1H,ddd ,J=2.3,8.8,8.8Hz) ,7.57 (1H,d,J=8.5Hz) ,7.81 (1H,dd,J=2.3,9 .9Hz) ,8.09 (1H,dd,J=5.0,8.8Hz) ,8.18 (1H,dd,J=2.0,8.5Hz) , 8.42 (1H,d,J=2.0Hz) ,8.51 (1H,s) .
IR νₘₐₓ (KBr) : 3448,3233,3104,3027,2924,1711,1607,1516,14 61,1352,1306,1217,1153,1137 cm⁻¹.

### Example 17

### 5-Fluoro-N-[2-methanesulfonyl-4-(2-methylthiazol-4-yl)phenyl]-3-methylbenzo[b]thiophene-2-sulfonamide (Compound 17)

Into 4.0 mL of chloroform was dissolved 200 mg of N-(4-acetyl-2-methanesulfonylphenyl)-5-fluoro-3-methylbenzo[b]thiophene-2-sulfonamide, and 194 mg of benzyltrimethylammonium tribromide was added, followed by 1 hour of stirring at room temperature. The reaction was terminated by adding water to the reaction solution and then the solvent was once removed by evaporation. The residue was adjusted to pH 2 to 3 by adding 1 mol/L hydrochloric acid, followed by extraction with ethyl acetate. The organic layer was washed with saturated brine and then dried over anhydrous sodium sulfate. The solvent was removed by evaporation under reduced pressure and the resulting residue was washed with methanol to obtain 201 mg of N-(4-bromoacetyl-2-methanesulfonylphenyl)-5-fluoro-3-methylbenzo[b]thiophene-2-sulfonamide as a colorless solid. The thus obtained N-(4-bromoacetyl-2-methanesulfonylphenyl)-5-fluoro-3-methylbenzo[b]thiophene-2-sulfonamide (150 mg) was dissolved into a mixed solution of 1.5 mL of dioxane and 1.5 mL of ethanol, and 53 mg of sodium hydrogen carbonate and 26 mg of thioacetamide were successively added, followed by 2 hours of stirring under heating and refluxing. The reaction was terminated by adding water to the reaction solution and then the solvent was removed by evaporation under reduced pressure. The residue was adjusted to pH 2 to 3 by adding 1 mol/L hydrochloric acid, followed by extraction with ethyl acetate. The organic layer was washed with saturated brine and then dried over anhydrous sodium sulfate. The solvent was removed by evaporation under reduced pressure and the resulting residue was purified by silica gel column chromatography to obtain 61 mg of the title compound as light yellow powder.
Melting point: 217-220°C
¹H-NMR (CDCl₃) : δ 2.68 (3H,s) , 2.75 (3H, s) , 2.99 (3H, s ) 7.27(1H, ddd, J=2.4, 8.7, 9.0Hz), 7.33(1H, s), 7.45 (1H, dd, J=2.4, 9.3Hz) , 7.75 (1H, dd J=4.5, 9.0Hz) , 7.84 (1H, d, J=8.7Hz) , 8.06(1H, dd, J=2.1, 8.7Hz) , 8.35(1H, d, J= 2.1Hz), 9.55(1H, s).
IR νₘₐₓ (KBr) : 3237,1604,1514,1352,1296,1163,899 cm⁻¹.

### Example 18

### 5-Fluoro-N-[2-methanesulfonyl-4-((E)-2-methanesulfinyl-2-methylsulfanylvinyl)phenyl]-3-methylbenzo[b]thiophene-2-sulfonamide (Compound 18)

Under an argon atmosphere, 135 mg of the compound of Reference Example 6 was dissolved into 2 mL of THF, and 196 mg of methyl methylsulfinylmethyl sulfide and 862 µL of 40% methanol solution of Triton B were added successively, followed by 16 hours of stirring under heating and refluxing. After the reaction was terminated by adding 1 mol/L hydrochloric acid to the reaction solution, the mixture was diluted with ethyl acetate and the solution was washed with water and saturated brine, successively. After the solution was dried over anhydrous sodium sulfate, the solvent was removed by evaporation under reduced pressure and the resulting residue was purified by silica gel column chromatography (ethyl acetate/hexane = 1/1) to obtain 105 mg of the title compound as colorless amorphous.
¹H-NMR (CDCl₃) : δ 2.32 (3H,s) , 2.70 (3H, s) , 2.76(3H, s) , 3.04(3H,s), 7.28(1H, ddd, J=2.4, 9.0, 9.3Hz) , 7.48 (1H, dd, J=2.4, 9.3Hz) ,7.52(1H, s), 7.78 (1H, dd J=4.8, 9.0Hz ) , 7.83 (1H, d, J=8.7Hz) , 8.02(1H, dd, J=2.1, 8.7Hz) , 8. 46(1H, d, J=2.1Hz), 9.65(1H, s) .
IR νₘₐₓ (KBr) : 3446, 3225, 1604, 1492, 1303, 1163, 1133 , 924 cm⁻¹.

The following show instrumental data in each Example.

**Table 3**

| Example | Melting Point (°C) | H¹-NMR(δ) | | IR (νcm⁻¹, KBr) |
|---|---|---|---|---|
| 4 | 207-208 | CDCl₃ | 2.09 (3H, s), 2.69 (3H, s), 3.00 (3H, s), 3.98 (3H, s), 7.46 (1H, dd, J=1.9, 8.7Hz), 7.73 (1H, d, J=8.7Hz), 7.78 (1H, d, J=1.9Hz), 7.85 (1H, d, J=8.7Hz), 8.07 (1H, dd, J=2.1, 8.7Hz), 8.33 (1H, d, J=2.1Hz), 9.64 (1H, s). | 3423, 3021, 2931, 1658, 1482, 1351, 1296, 1281, 1163, 991, 929, 653 |
| 5 | 243-245 | CDCl₃ | 2.13 (3H, s), 2.30 (3H, s), 2.68 (3H, s), 3.01 (3H, s), 6.83 (1H, d, J=8.7Hz), 7.27 (1H, ddd, J=2.4, 8.7, 9.0Hz), 7.46 (1H, dd, J=2.4, 9.0Hz), 7.75 (1H, dd, J=4.5, 9.0Hz), 7.87 (1H, d, J=8.7Hz), 8.16 (1H, dd, J=2.1, 8.7Hz), 8.42 (1H, d, J=2.1Hz), 9.66 (1H, s). | 3218, 1641, 1495, 1295, 1163, 913 |
| 6 | amorphous | DMSO-d₆ | 2.38 (3H, s), 2.59 (3H, s), 3.34 (3H, s), 7.04 (1H, s), 7.48 (1H, ddd, J=2.5, 8.9, 9.2Hz), 7.60 (1H, d, J=8.6Hz), 7.84 (1H, dd, J=2.5, 9.9Hz), 8.12 (1H, dd, J=5.0, 8.9Hz), 8.18 (1H, dd, J=2.1, 8.6Hz), 8.36 (1H, d, J=2.1Hz). | 3161, 3089, 3000, 2912, 1653, 1613, 1498, 1308, 1156, 908, 647 |
| 7 | 176-179 | CDCl₃ | 1.39 (3H, t, J=6.9Hz), 2.09 (3H, s), 2.68 (3H, s), 2.99 (3H, s), 4.22 (2H, q, J=2.1Hz), 7.27 (1H, ddd, J=2.4, 8.7, 9.0Hz), 7.46 (1H, dd, J=2.4, 9.0Hz), 7.75 (1H, dd, J=4.5, 9.0Hz), 7.86 (1H, d, J=8.7Hz), 8.08 (1H, dd, J=2.1, 8.7Hz), 8.33 (1H, d, J=2.1Hz), 9.66 (1H, s). | 3220, 1660, 1481, 1297, 1161, 923 |
| 8 | amorphous | CDCl₃ | 2.35 (3H, s), 2.38 (3H, s), 2.68 (3H, s), 3.01 (3H, s), 7.27 (1H, ddd, J=2.4, 8.6, 8.8Hz), 7.46 (1H, dd, J=2.4, 9.2Hz), 7.75 (1H, dd, J=4.7, 8.8Hz), 7.83 (1H, d, J=8.7Hz), 8.01 (1H, dd, J=2.1, 8.7Hz), 8.32 (1H, d, J=2.1Hz), 9.61 (1H, s). | 3228, 3060, 3023, 2923, 1606, 1507, 1442, 1355, 1304, 1163, 1136, 925, 652 |
| 12 | 242-244 | DMSO-d₆ | 2.44 (3H, s), 3.21 (3H, s), 4.28 (2H, s), 7.31 (1H, ddd, J=2.0, 9.0, 9.0Hz), 7.46 (1H, d, J=8.6Hz), 7.65 (1H, dd, J=2.0, 8.0Hz), 7.89 (1H, s), 7.95 (1H, dd, J=4.8, 9.0Hz), 7.98 (1H, d, J=8.6Hz), 8.24 (1H, s). | 3221, 3132, 2927, 1615, 1520, 1484, 1355, 1341, 1304, 1100 |
| 13 | 197-199 | CDCl₃ | 2.69 (3H, s), 3.03 (3H, s), 4.53 (2H, s), 7.27 (1H, ddd, J=2.4, 8.6, 8.6Hz), 7.47 (1H, dd, J=2.4, 9.2Hz), 7.70 (1H, s), 7.75 (1H, dd, J=4.8, 8.6Hz), 7.92 (1H, d, J=8.8Hz), 8.20 (1H, dd, J=2.0, 8.8Hz), 8.49 (1H, d, J=2.0Hz), 9.72 (1H, s). | 3228, 2927, 1613, 1560, 1516, 1484, 1440, 1398, 1373, 1357, 1304, 1250, 1194, 1162, 1099 |
| 14 | 206-208 | CDCl₃ | 2.22 (3H, s), 2.69 (3H, s), 3.02 (3H, s), 7.28 (1H, ddd, J=2.4, 9.0, 9.0Hz), 7.42 (1H, d, J=2.0Hz), 7.46 (1H, dd, J=2.4, 9.2Hz), 7.75 (1H, dd, J=4.8, 9.0Hz), 7.89 (1H, d, J=8.6Hz), 8.17 (1H, dd, J=2.0, 8.6Hz), 8.47 (1H, d, J=2.0Hz), 9.70 (1H, s). | 3231, 3131, 2925, 1614, 1519, 1486, 1439, 1355, 1332, 1302, 1275, 1161, 1138, 1099 |

Then, chymase inhibitory activity was tested on the representative compounds of the invention in accordance with the following Test Example.

### Test Example 1

### Measurement of inhibitory activity of human chymase

Human chymase used was obtained from silkworms infected with baculovirus integrated with a gene encoding human chymase (FEBS. Let., 412, 86 (1997)).

Chymase activity was determined with reference to the method known in a literature (Miyazaki et al., Kekkan, Vol. 20, p. 207 (1997)). That is, the activity was measured by reacting free His-Leu formed together with Ang II with o-phthalaldehyde (hereinafter, abbreviated as OPT) to prepare a fluorescent derivative and determining the amount quantitatively by means of a fluorophotometer.

First, 3.6 µmol of each test compound was weighed in a test tube and was dissolved into 3 mL of DMSO. The DMSO solution was diluted 1000-fold with 20 mmol/L Trishydrochloric acid buffer solution (pH 8.0) containing 0.01% Triton X-100 and 0.5 mol/L potassium chloride to prepare 1.2×10⁻⁶ mol/L solution, which was successively diluted with the buffer solution to prepare test sample solutions having concentrations of 1.2×10⁻⁶ mol/L to 1.2×10⁻⁹ mol/L. To 500 µL of the test sample solution of each concentration or buffer solution was added 50 µL of an enzyme solution, followed by 10 minutes of preincubation at 37°C. Then, 50 µL of 0.1 mmol/L Ang I solution was added to initiate a reaction. Human angiotensin I (manufactured by SIGMA) was employed as Ang I. The enzyme solution to be used for the reaction was adjusted so as to hydrolyze about 60% of substrate under the conditions, and the reaction wherein a buffer solution containing no enzyme was carried out as a blind test. After 120 minutes of incubation at 37°C, the reaction was terminated by adding 900 µL of 15 % of trichloroacetic acid. Thereafter, the reaction mixture was centrifuged at 4°C at 3,000 rpm for 10 minutes and 2 mL of 2 mol/L sodium hydroxide and 1 mL of methanol were added to 1 mL of the resulting supernatant. Thereto was added 100 µL of methanol solution containing 1.2 mg of N-acetyl-L-cysteine and 1 mg of OPT per 1 mL, whereby a derivatization reaction was initiated. After the reaction mixture was left on standing for exactly 1 hour, fluorescence intensity at fluorescence wavelength of 502 nm under excitation wavelength of 304 nm was measured. The measurement was repeated twice for each sample and blind test. The fluorescence intensity obtained by subtracting the average value at blind test from the average value thereof was determined as chymase activity.

In this regard, an enzymatic reaction using a buffer solution instead of the test sample solution was carried out as a control, and inhibitory ratio of chymase activity was determined as percentage by dividing the difference of subtraction of the activity at the addition of the test compound from the chymase activity at the control by the chymase activity at the control. Based on each inhibitory ratio, the concentration at which 50% of the activity was inhibited (hereinafter, referred to as IC₅₀ value) was calculated. Table 4 shows inhibitory ratio at 10⁻⁷ mol/L (%) and IC₅₀ values of human chymase of representative compounds.

**Table 4**

| | | |
|---|---|---|
| Inhibitory Activity of Compounds | | |

| Compound | Inhibitory ratio at 10⁻⁷ mol/L (%) | IC₅₀ value (nmol/L) |
|---|---|---|
| Compound 1 | 65.3 | |
| Compound 2 | 68.2 | 102 |
| Compound 3 | 55.6 | |
| Compound 5 | 70.1 | |
| Compound 6 | 78.0 | |
| Compound 9 | 85.3 | 20 |
| Compound 11 | 50.1 | |
| Compound 12 | 79.8 | 39 |
| Compound 13 | 53.9 | |
| Compound 14 | 63.3 | 101 |
| Compound 16 | 90.7 | 7 |
| Compound 18 | 88.1 | 20 |

### Industrial Applicability

The N-substituted benzothiophenesulfonamide derivatives or pharmaceutically acceptable salts thereof of the invention have a selective inhibitory action on chymase and are useful as agents for preventing or treating cardiac and circulatory diseases, especially cardiac infarction, restenosis after PTCA and intimal thickening after bypass grafting caused by abnormal increase of production of angiotensin II or endothelin I based on chymase activity, or by activation of mast cell.

## Claims

1. An N-substituted benzothiophenesulfonamide derivative represented by the formula (I): 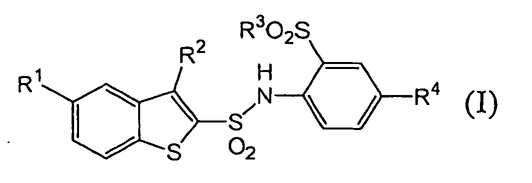 wherein R¹ represents a hydrogen atom, a halogen atom or a lower alkyl group having 1 to 4 carbon atoms; R² and R³ each may be the same or different and represents a lower alkyl group having 1 to 4 carbon atoms; and R⁴ represents a monocyclic heterocyclic group represented by the formula: 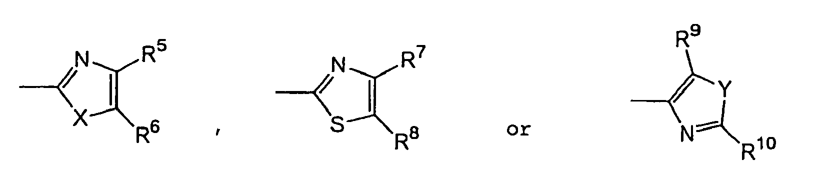 in which X represents an oxygen atom or NH, Y represents an oxygen atom, a sulfur atom or NH, R⁵ and R⁶ each may be the same or different and represents a hydrogen atom, a lower alkyl group having 1 to 4 carbon atoms which may be substituted by a halogen atom, a lower alkoxy group having 1 to 4 carbon atoms or a hydroxy lower alkyl group having 1 to 4 carbon atoms, R⁷ and R⁸ each may be the same or different and represents a hydrogen atom, a lower alkyl group having 1 to 4 carbon atoms which may be substituted by a halogen atom, a lower alkoxy group having 1 to 4 carbon atoms, a hydroxy lower alkyl group having 1 to 4 carbon atoms, a lower alkoxycarbonyl group having 1 to 4 carbon atoms in the alkoxy residue or a carboxyl group, R⁹ and R¹⁰ each may be the same or different and represents a hydrogen atom, a lower alkyl group having 1 to 4 carbon atoms which may be substituted by a halogen atom, a lower alkoxy group having 1 to 4 carbon atoms, or a hydroxy lower alkyl group having 1 to 4 carbon atoms, provided that R⁵ and R⁶, R⁷ and R⁸, and R⁹ and R¹⁰ each are not hydrogen atoms at the same time, or
a group represented by the formula: 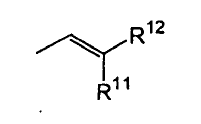 in which R¹¹ and R¹² each may be the same or different and represents a hydrogen atom, a lower alkyl group having 1 to 4 carbon atoms, a lower alkylsulfanyl group having 1 to 4 carbon atoms, a lower alkylsulfinyl group having 1 to 4 carbon atoms, a lower alkylsulfonyl group having 1 to 4 carbon atoms or a lower alkoxycarbonyl group having 1 to 4 carbon atoms in the alkoxy residue, or a pharmaceutically acceptable salt thereof.

2. The N-substituted benzothiophenesulfonamide derivative according to claim 1, wherein said derivative or a pharmaceutically acceptable salt thereof is selected from the group consisting of 2-[4-(5-fluoro-3-methylbenzo[b]thiophene-2-sulfonylamino)-3-methanesulfonylphenyl]thiazole-4-carboxylic acid, 5-fluoro-N-[4-(4-hydroxymethylthiazol-2-yl)-2-methanesulfonylphenyl]-3-methylbenzo[b]thiophene-2-sulfonamide, 5-fluoro-N-[2-methanesulfonyl-4-(5-methoxy-4-methyloxazol-2-yl)phenyl]-3-methylbenzo[b]thiophene-2-sulfonamide, 5-fluoro-N-[2-methanesulfonyl-4-(5-methyloxazol-2-yl)phenyl]-3-methyl-benzo[b]thiophene-2-sulfonamide and 5-fluoro-N-[2-methanesulfonyl-4-((E)-2-methanesulfinyl-2-methylsulfanylvinyl)phenyl]-3-methylbenzo[b]thiophene-2-sulfonamide.

3. A chymase inhibitor comprising the N-substituted benzothiophenesulfonamide derivative or a pharmaceutically acceptable salt thereof as defined in claim 1 or 2.

4. A medicine comprising the N-substituted benzothiophenesulfonamide derivative or a pharmaceutically acceptable salt thereof as defined in claim 1 or 2.

## Patentansprüche

1. N-substituiertes Benzothiophensulfonamidderivat, dargestellt durch die Formel (I): 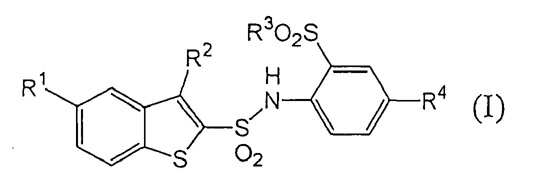 wobei R¹ ein Wasserstoffatom darstellt, ein Halogenatom oder eine Niedrigalkylgruppe mit 1 bis 4 Kohlenstoffatomen; R² und R³ können jeweils gleich oder verschieden sein und stellen eine Niedrigalkylgruppe mit 1 bis 4 Kohlenstoffatomen dar; und R⁴ stellt eine monocyclische heterocyclische Gruppe dar, dargestellt durch die Formel: 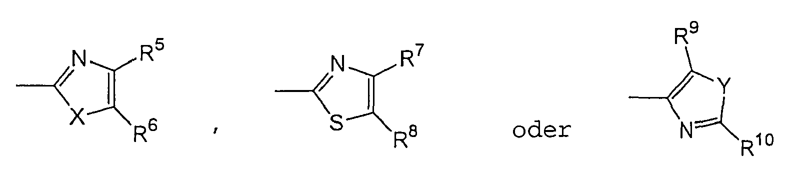 wobei X ein Sauerstoffatom oder NH darstellt, Y ein Sauerstoffatom, ein Schwefelatom oder NH darstellt, R⁵ und R⁶ jeweils gleich oder verschieden sein können und ein Wasserstoffatom, eine Niedrigalkylgruppe mit 1 bis 4 Kohlenstoffatomen, die mit einem Halogenatom substituiert sein kann, eine Niedrigalkoxygruppe mit 1 bis 4 Kohlenstoffatomen oder eine Hydroxy-Niedrigalkylgruppe mit 1 bis 4 Kohlenstoffatomen darstellen, R⁷ und R⁸ können jeweils gleich oder verschieden sein und stellen ein Wasserstoffatom, eine Niedrigalkylgruppe mit 1 bis 4 Kohlenstoffatomen, welche substituiert sein kann mit einem Halogenatom, eine Niedrigalkoxygruppe mit 1 bis 4 Kohlenstoffatomen, eine Niedrigalkylhydroxygruppe mit 1 bis 4 Kohlenstoffatomen, eine Niedrigalkoxygruppe mit 1 bis 4 Kohlenstoffatomen im Alkoxyrest oder eine Carboxylgruppe dar, R⁹ und R¹⁰ können jeweils gleich oder verschieden sein und stellen ein Wasserstoffatom, eine Niedrigalkylgruppe mit 1 bis 4 Kohlenstoffatomen, welche mit einem Halogenatom substituiert sein kann, eine Niedrigalkoxygruppe mit 1 bis 4 Kohlenstoffatomen oder eine Hydroxy-Niedrigalkylgruppe mit 1 bis 4 Kohlenstoffatomen dar, vorausgesetzt, dass R⁵ und R⁶, R⁷ und R⁸ und R⁹ und R¹⁰ jeweils gleichzeitig Wasserstoffatome sind oder eine Gruppe, dargestellt durch die Formel: 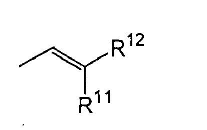 wobei R¹¹ und R¹² jeweils gleich oder verschieden sein können und ein Wasserstoffatom, eine Niedrigalkylgruppe mit 1 bis 4 Kohlenstoffatomen, eine Niedrigalkylsulfanylgruppe mit 1 bis 4 Kohlenstoffatomen, eine Niedrigalkylsulfinylgruppe mit 1 bis 4 Kohlenstoffatomen, eine Niedrigalkylsulfonylgruppe mit 1 bis 4 Kohlenstoffatomen oder eine Niedrigalkoxycarbonylgruppe mit 1 bis 4 Kohlenstoffatomen im Alkoxyrest darstellen, oder ein pharmazeutisch verträgliches Salz davon.

2. N-substituiertes Benzothiophensulfonamidderivat gemäß Anspruch 1, wobei das Derivat oder ein pharmazeutisch verträgliches Salz davon ausgewählt ist aus der Gruppe, bestehend aus 2-[4-(5-Fluor-3-methylbenzo[b]thiophen-2-sulfonylamino)-3-methansulfonylphenyl]thiazol-4-carbonsäure, 5-Fluor-N-[4-(4-hydroxymethylthiazol-2-yl)-2-methansulfonylphenyl]-3-methylbenzo[b]thiophen-2-sulfonamid, 5-Fluor-N-[2-methansulfonyl-4-(5-methoxy-4-methyloxazol-2-yl)phenyl]-3-methylbenzo[b]thiophen-2-sulfonamid, 5-Fluor-N-[2-methansulfonyl-4-(5-methyloxazol-2-yl)phenyl]-3-methylbenzo[b]thiophen-2-sulfonamid und 5-Fluor-N-[2-methansulfonyl-4-((E)-2-methansulfinyl-2-methylsulfanylvinyl)phenyl]-3-methylbenzo[b]thiophen-2-sulfonamid.

3. Chymaseinhibitor, umfassend das N-substituierte Benzothiophensulfonamidderivat oder ein pharmazeutisch verträgliches Salz davon, wie in Anspruch 1 oder 2 definiert.

4. Arzneimittel, umfassend das N-substituierte Benzothiophensulfonamidderivat oder ein pharmazeutisch verträgliches Salz davon, wie in Anspruch 1 oder 2 definiert.

## Revendications

1. Dérivé de benzothiophènesulfonamide N-substitué représenté par la formule (I) : 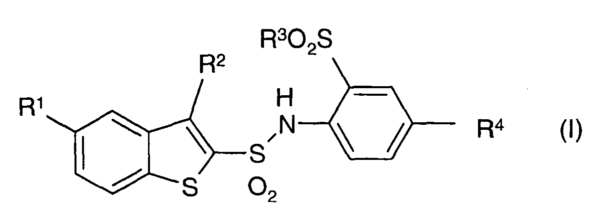 dans laquelle R¹ représente un atome d'hydrogène, un atome d'halogène ou un groupe alkyle inférieur ayant de 1 à 4 atomes de carbone ; R² et R³ peuvent être identiques ou différents et représentent chacun un groupe alkyle inférieur ayant de 1 à 4 atomes de carbone ; et R⁴ représente un groupe hétérocyclique monocyclique représenté par les formules : 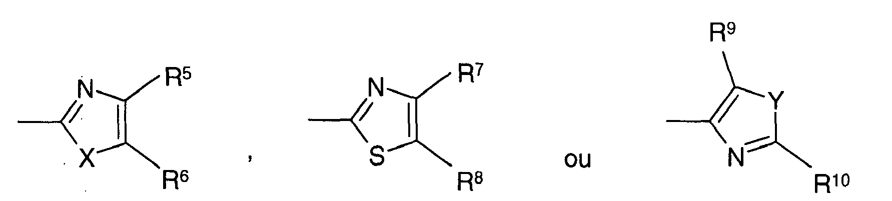 dans lesquelles X représente un atome d'oxygène ou NH, Y représente un atome d'oxygène, un atome de soufre ou NH, R⁵ et R⁶ peuvent être identiques ou différents et représentent chacun un atome d'hydrogène, un groupe alkyle inférieur ayant de 1 à 4 atomes de carbone qui peut être substitué par un atome d'halogène, un groupe alcoxy inférieur ayant de 1 à 4 atomes de carbone ou un groupe hydroxyalkyle inférieur ayant de 1 à 4 atomes de carbone, R⁷ et R⁸ peuvent être identiques ou différents et représentent chacun un atome d'hydrogène, un groupe alkyle inférieur ayant de 1 à 4 atomes de carbone qui peut être substitué par un atome d'halogène, un groupe alcoxy inférieur ayant de 1 à 4 atomes de carbone, un groupe hydroxyalkyle inférieur ayant de 1 à 4 atomes de carbone, un groupe alcoxycarbonyle inférieur ayant de 1 à 4 atomes de carbone dans le résidu alcoxy ou un groupe carboxyle, R⁹ et R¹⁰ peuvent être identiques ou différents et représentent chacun un atome d'hydrogène, un groupe alkyle inférieur ayant de 1 à 4 atomes de carbone qui peut être substitué par un atome d'halogène, un groupe alcoxy inférieur ayant de 1 à 4 atomes de carbone, ou un groupe hydroxyalkyle inférieur ayant de 1 à 4 atomes de carbone, à condition que R⁵ et R⁶, R⁷ et R⁸, et R⁹ et R¹⁰ ne soient pas simultanément des atomes d'hydrogène, ou
un groupe représenté par la formule : 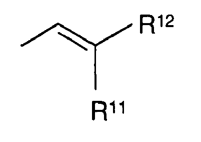 dans laquelle R¹¹ et R¹² peuvent être identiques ou différents et représentent chacun un atome d'hydrogène, un groupe alkyle inférieur ayant de 1 à 4 atomes de carbone, un groupe alkylsulfanyle inférieur ayant de 1 à 4 atomes de carbone, un groupe alkylsulfinyle inférieur ayant de 1 à 4 atomes de carbone, un groupe alkylsulfonyle inférieur ayant de 1 à 4 atomes de carbone ou un groupe alcoxycarbonyle inférieur ayant de 1 à 4 atomes de carbone dans le résidu alcoxy, ou sel pharmaceutiquement acceptable de celui-ci.

2. Dérivé de benzothiophènesulfonamide N-substitué selon la revendication 1, dans lequel ledit dérivé ou sel pharmaceutiquement acceptable de celui-ci est choisi dans le groupe constitué de l'acide 2-[4-(5-fluoro-3-méthylbenzo[b]thiophène-2-sulfonylamino)-3-méthanesulfonylphényl]thiazole-4-carboxylique, le 5-fluoro-N-[4-(4-hydroxyméthylthiazol-2-yl)-2-méthane-sulfonylphényl]-3-méthylbenzo[b]thiophène-2-sulfonamide, le 5-fluoro-N-[2-méthanesulfonyl-4-(5-méthoxy-4-méthyloxazol-2-yl)phényl]-3-méthylbenzo[b]thiophène-2-sulfonamide, le 5-fluoro-N-[2-méthanesulfonyl-4-(5-méthyloxazol-2-yl)phényl]-3-méthylbenzo[b]-thiophène-2-sulfonamide et le 5-fluoro-N-[2-méthanesulfonyl-4-((E)-2-méthanesulfinyl-2-méthylsulfanylvinyl)phényl]-3-méthylbenzo[b]thiophène-2-sulfonamide.

3. Inhibiteur de chymase comprenant le dérivé de benzothiophènesulfonamide N-substitué ou un sel pharmaceutiquement acceptable de celui-ci comme défini dans la revendication 1 ou 2.

4. Médicament comprenant le dérivé de benzothiophènesulfonamide N-substitué ou un sel pharmaceutiquement acceptable de celui-ci comme défini dans la revendication 1 ou 2.
